# EUROPEAN PATENT APPLICATION

(11) **EP 0 565 010 A1**
(43) Date of publication of application: **13.10.1993**
(21) Application number: 93105555.2
(22) Date of filing: 03.04.1993
(51) Int. Cl.: A61K 7/06, B05C 9/04

(54) **Hair dye and hair growth composition**

(30) Priority: 10.04.1992 IT PN920030
(71) Applicant: Rizzo, Antonio, I-30027 San Dona' di Piave, Venezia (IT)
(72) Inventor: Rizzo, Antonio, I-30027 San Dona' di Piave, Venezia (IT)
(74) Representative: Giugni, Valter

(57) **Abstract**

Composition for topical use having properties that are effective in stimulating new hair growth, reinvigorating existing hair, preventing hair from turning grey and promoting hair repigmentation. It comprises substances which are normally present in the human organism. As active substances, the composition in fact contains levodopa, phosphate creatine, ursodeoxycholic acid and *L*-ascorbic acid.

The composition is preferably prepared in the form of a lotion in single-dose phials, and it has no undesirable side-effect.

## Description

The present invention relates to a composition for topical use, which is effective in reinvigorating and stimulating new hair growth, preventing hair from turning grey, and promoting natural hair repigmentation, said composition containing strictly natural substances that are physiologically present in the human organism.

Various products are known to be currently marketed, which are claimed to be effective, to a greater or smaller extent, in solving the above cited hair-related problems.

The best-known ones among these products contain, as the active principle, minoxidil (6-amino-1,2-dihydro-1-hydroxy-2-imino-4-piperidine-pirimidine) by US-based Upjohn Co. In particular, the patent application WO 86/04231 can be cited here in this connection.

Minoxidil has proven effective in stimulating hair growth in persons affected with androgenic alopecia, while starting to show its effect after approx. 3 to 4 months of regular application. When its use is dicontinued, any new hair growth ceases almost immediately and, after a short while, the symtoms prevailing before the treatment appear again. The mechanism of action or, better, the way in which minoxidil works in the treatment of androgenic baldness is not known exactly. However, the use of minoxidil-based compositions may give rise to dermatological reactions, such as desquamation, erythemas, etc., or even to some allergic reaction. But, above all, there are indications of a risk of systemic effects, particularly hypotension.

The Japanese patent application no. J61227518-A filed to the name of Kaneko T., and laid to public inspection, discloses a composition for preventing hair from turning grey, which contains minoxidil and levodopa [2-amino-3(3,4-dihydroxyphenyl)propanoic acid; also known as β-(3,4-dihydroxyphenyl)-*d*-alanine; or simply *L*-dopa] as active principles.

According to said Japanese patent application, minoxidil, further to stimulate new hair growth, is effective also as an inhibitor of hair greying processes, the combination of monoxidil with levodopa being allegedly responsible for a magnification of said particular effect. No indication is on the other hand given on possible positive new hair growth effects attributable to the use of levodopa. Furthermore, the composition disclosed in said Japanese patent application still contains minoxidil, which is a synthetic substance, with all afore mentioned pharmacological contraindications. Its application cannot therefore occur indiscriminately and it is rather suggested that it be in all cases carried out under strict medical control.

It is a purpose of the present invention to provide a composition for topical use which proves to be actually effective in reinvigorating and stimulating new hair growth, preventing hair from turning grey and promoting natural hair repigmentation, while eliminating any negative side-effect, thanks to the use of natural substances that are physiologically present in the human organism, so as to enable any person to be indiscriminately treated therewith. Furthermore, the composition according to the present invention can be produced in a particularly simple and cost-effective way.

Substantially, the characteristics of the invention are as recited in the appended claims. Features and advantages of the present invention are however further described below, by way of non-limiting example, with reference to a preferred embodiment and some experimental and testing work done on the subject.

The new composition according to the invention has been studied starting from levodopa as its basis, since it has been ascertained that this substance alone has the ability of activating new growth of hair, further to promoting hair repigmentation, without on the other hand giving rise to any undesired side-effect.

As a matter of fact, levodopa, when applied directly to the scalp, performs a function of activation of the local microcirculation, through selective blood vessel dilation of the vascular system, by eliminating the excess of tissue calcium and magnesium that is responsible for folliculate anoxia.

Furthermore, levodopa promotes hair repigmentation in that it is a natural amino acid which is converted into melanin by means of biochemical transformation processes activated by an enzyme, ie. dopa oxidase, that is present in the skin.

On the other hand, a further purpose of the present invention was specifically to strengthen the action of levodopa by having it associated with other strictly natural substances that are capable of synergetically cooperating with levodopa so as to enhance the effects in the treatment of hair.

After a thorough experimental work, the inventor has succeeded in finding out that a second active principle, which is effective in strengthening said function of activation of the local microcirculation, while opposing a state of folliculate asthenia, consists of a phosphoric acid salt, such as preferably phosphocreatine, ie. a substance that is present in muscular tissues, of which it constitutes the reserve of energy. This substance combines readily with levodopa and the resulting composition has proven capable of performing a quicker, more intensive action in support of new hair growth and hair reinvigoration, as well as in terms of preventing hair from turning grey and promoting hair repigmentation.

Further experimental work highlighted the possibility of enhancing the effectiveness of the composition by further associating another strictly natural substance therewith, and namely a deoxycholic acid, ie. a bile acid known as a cholesterol antagonist, which has proven capable of performing an action of activation of local microcirculation and skin oxygenation, while opposing retention and stagnation of lipids. In a preferred way, ursodeoxycholic acid is used in this connection, since it appears to be particularly effective and favourable in view of its application for the treatment of persons afflicted with an excess of scalp sebum.

Finally, the inventor has observed that the new composition proposed by him, when prepared in multiple doses to be used at successive intervals of time, would be subject to degradation owing to levadopa undergoing a decarboxylation process. It therefore appears appropriate to complete the composition with the addition of a decarboxylase inhibitor; the preferred substance in this connection is *L*-ascorbic acid, since it is a natural, readily available and economical substance, which therefore acts as a preservative, while also contributing to the activation of local microcirculation.

It has been demonstrated experimentally that the new composition is actually capable of enabling four important results to be achieved, ie.: stimulating new hair growth, reinvigorating existing hair, preventing hair from turning grey, and promoting natural hair repigmentation, wherein it will be appreciated that new hair growth may of course only take place if follicles are not definitively atrophied.

In various patients treated under medical control, the new composition has demonstrated to be entirely free of undesirable side-effects. Even in the case of repeated application in too large a dose, as the inventor himself has been able to ascertain after long-term testing on his person, no adverse reactions are to be expected.

A further advantage of the composition according to the present invention lies in the really very short time taken by the positive effects of the treatment to show up. These in fact take just a few weeks, and not months as it is usually the case for similar products being currently marketed, from the beginning of the treatment to be fully noticeable. And, with constant use, said positive effects become persisting.

In a preferred way, the new composition is prepared in the form of a lotion for topical use, containing, per each 100 ml of lotion: 1) 2,500 mg of levodopa; 2) 500 mg of phosphate creatine; 3) 600 mg of ursodeoxycholic acid; 120 mg of *L*-ascorbic acid; under addition of distilled water and ethyl alcohol at 95°, preferably in equal portions, up to 100 ml. Alcohol should be added at the end of the preparation process, after all other substances involved will have been dissolved in the distilled water.

The inventor has on the other hand also experimented with different doses than the afore described one, obtaining however substantially equivalent results. In particular, the quantity of levodopa has been varied from 1,500 to 4,500 mg, and each quantity of phosphate creatine and ursodeoxycholic acid has been varied from 10 percent and 30 percent of the quantity of levodopa; the quantity of *L*-ascorbic acid cannot on the contrary be varied to any level in excess of 5 percent of the quantity of levodopa, since the hair repigmentation effect would in that case turn excessive and no longer controllable.

Starting from its first formulation, the lotion has been tested, under medical control, in a number of persons, including the inventor himself, and has immediately given favourable results, some of them are listed below for due reference.

In a 66-year old man afflicted with aerated alopecia, the beginning of a resolution of the pathology has already been observed after just 40 days of treatment.

In a 28-year old woman afflicted with aerated alopecia, complete healing has been observed after 120 days of treatment.

Twelve men and a woman aged between 35 and 65 years, including the inventor himself, who were afflicted with different pathologies varying from simple hair loss to androgenic alopecia, have been observed to achieve extremely favourable results after a period of 40 to 60 days, with a peak of 120 days of treatment.

The lotion may of course be enriched through the addition of any desired scent to any desired quantity, much in the same way as it is usually done in products of the same kind.

Taking into due account the fact that the solution is subject to oxidation owing to the presence of levodopa, the lotion should most adequately be prepared in 5-ml or 7-ml or 10-ml single-dose phials, according to the extent of the zone of the scalp that has to be treated.

The procedure for the application of the lotion require it to be applied on a daily basis, preferably in the morning, by shortly and slightly rubbing the scalp therewith under dry hair conditions. No preliminary hair shampooing is required.

When there are no acute hair loss problems, the lotion may be used at longer time intervals, even on a weekly basis, without impairing the beneficial effects thereof.

It will be appreciated that the afore described composition may be the subject of any modification as considered appropriate in percentage terms, as long as the presence of at least all of the afore mentioned active substances is ensured, and that it may also be produced in the form of a cream or a gel, instead of a lotion, by adding any well-known and commonly used substance to that purpose, without departing from the scopes of the present invention.

## Claims

1. Composition for topical use having properties capable of stimulating new hair growth, reinvigorating existing hair, preventing hair from turning grey and promoting hair repigmentation, comprising levodopa as an active substance, **characterized in that** it further contains a phosphoric acid salt, in a quantity situated anywhere between 10 and 30 percent of the quantity of levodopa, so as to strengthen the activation of the local microcirculation, and an inhibitor of decarboxylase, in no higher quantity than 5 percent of the quantity of levodopa, so as to prevent the composition from spoiling.

2. Composition according to claim 1, **characterized in that** said phosphoric acid salt consists of phosphate creatine.

3. Composition according to claim 1, **characterized in that** said inhibitor of decarboxylase consists of *L*-ascorbic acid.

4. Composition according to claim 1, **characterized in that** it further contains a deoxycholic acid to a quantity situated anywhere between 10 and 30 percent of the quantity of levodopa, so as to remove the excess of scalp sebum.

5. Composition according to claim 4, **characterized in that** said deoxycholic acid consists of ursodeoxycholic acid.

6. Composition according to any of the previous claims, **characterized in that** in the preparation in the form of a lotion the quantities of said active substances in 100 ml of lotion are as follows: 2,500 mg of levodopa; 500 mg of phosphate creatine; 600 mg of ursodeoxycholic acid; 120 mg of *L*-ascorbic acid, under addition of distilled water and ethyl alcohol at 95°, in equal portions, as well as scenting substances, up to 100 ml.
